# EUROPÄISCHE PATENTSCHRIFT

(11) **EP 0 557 336 B1**
(45) Veröffentlichungstag und Bekanntmachung des Hinweises auf die Patenterteilung: **03.05.1995**
(21) Anmeldenummer: 91919694.9
(22) Anmeldetag: 14.11.1991
(51) Int. Cl.: A61K 31/54, C07D 285/24

(54) **VERWENDUNG VON 2H-1,2,4-BENZOTHIADIAZIN-3(4H)-THION-1,1-DIOXIDEN ALS ANTIVIRALE ARZNEIMITTEL**
USE OF 2H-1,2,4-BENZOTHIADIAZINE-3(4H)-THION-1,1-DIOXIDES AS ANTIVIRAL MEDICAMENTS
UTILISATION DE 2H-1,2,4-BENZOTHIADIAZINO-3(4H)-THIONO-1,1-DIOXYDES COMME MEDICAMENTS ANTIVIRAUX

(30) Priorität: 16.11.1990 DE 4036571
(43) Veröffentlichungstag der Anmeldung: 01.09.1993
(73) Patentinhaber: BOEHRINGER MANNHEIM GMBH, 68298 Mannheim (DE)
(72) Erfinder: KÖNIG, Bernhard, D-8137 Berg 3 (DE); SEIDEL, Hans, D-8132 Tutzing (DE); LESER, Ulrike, D-8000 München 70 (DE); LEINERT, Herbert, D-6148 Heppenheim (DE); ZILCH, Harald, D-6800 Mannheim 31 (DE); MERTENS, Alfred, D-6905 Schriesheim (DE)
(74) Vertreter: Köster, Reinhold, Dr.
(86) Internationale Anmeldenummer: EP9102146
(87) Internationale Veröffentlichungsnummer: WO9208462

(56) Entgegenhaltungen:
- FR-A- 4 279
- Patent Abstracts of Japan, volume 9, No. 241 (C-306) (1964) 27 September 1985;

## Beschreibung

Gegenstand der vorliegenden Erfindung ist die neue Verwendung von 2H-1,2,4-Benzothiadiazin-3(4H)-thion-1,1-dioxiden als antivirale Arzneimittel.

In der Anmeldung JP 60 97,970 ist u.a. das 2-Propyl-2H-1,2,4-benzothiadiazin-3(4H)-thion-1,1-dioxid mit fungizider Wirkung beschrieben.

Das 6-Chlor-2H-1,2,4-benzothiadiazin-3(4H)-thion-1,1-dioxid ist in der U.S. 3,838,119 als Ausgangsmaterial zur Herstellung von Dihydrofurano[2',3':4,5]thiazolo-[2,2-c]-[1,2,4]benzothiadiazin-Derivaten eingesetzt worden.

Weiterhin ist das 2-(2-Methylphenyl)-2H-1,2,4-benzothiadiazin-3(4H)-thion-1,1-dioxid aus der Anmeldung Fr. M4279 mit tranquilizer, hypnotischer und hypotensiver Wirkung bekannt.

In weiteren Publikationen konnten für diese Substanzklasse eine cardiovaskuläre Wirkung (Pharmazie, 43, 37, 1988), eine antiinflammatorische Aktivität (Pharmazie, 44, 601, 1989; Boll.Chim.Farm., 126, 239, 1987), eine antihypertensive und mögliche diabetogene Wirkung (Arzneimittelforschung 31, 279 1981) und antimikrobielle Eigenschaften (Farmaco, Ed.Sci., 34, 189, 1979; dito, 34, 81, 1979; dito, 411, 1973; dito, 27, 990, 1972; dito, 38, 366, 1983) nachgewiesen werden.

Die Synthese dieser Substanzklasse ist u.a. in Farmaco, Ed.Sci., 38, 466, 1983 und in J.Med.Chem. 19, 524, 1976 beschrieben.

Die vorliegende Erfindung betrifft die neue Verwendung von Verbindungen der allgemeinen Formel I, 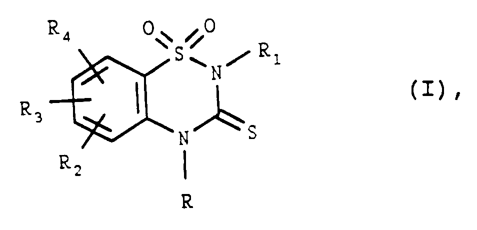
in der
- R₁: ein Wasserstoffatom, einen C₁-C₆-Alkylrest oder einen gegebenenfalls durch C₁-C₆-Alkyl substituierten Phenylrest bedeutet,
- R₂-R₄: gleich oder verschieden sind und unabhängig voneinander ein Wasserstoff- oder Halogenatom, einen C₁-C₆-Alkyl-, C₁-C₆-Alkoxy-, Trifluormethyl- oder Aminosulfonylrest und
- R: ein Wasserstoffatom, eine C₁-C₆-Alkyl- oder Phenyl-C₁-C₆-alkylgruppe bedeuten,
oder deren Tautomere zur Herstellung von Arzneimitteln zur Behandlung von viralen oder retroviralen Infektionen.

Die "C₁-C₆-Alkylteile" in der Definition der Alkyl- oder Alkoxygruppen können geradkettig oder verzweigt sein, wie z. B. die Methyl-, Ethyl-, Isopropyl-, n-Butyl-, Isobutyl- oder Hexylgruppe. Halogen bedeutet Fluor, Chlor, Brom oder Jod, vorzugsweise Chlor oder Brom. Phenyl-C₁-C₆-alkylgruppen sind beispielsweise die Benzyl- oder Phenylethylgruppe.

Insbesondere werden für die Herstellung von Arzneimitteln mit antiviraler oder antiretroviraler Wirkung solche Verbindungen der Formel II 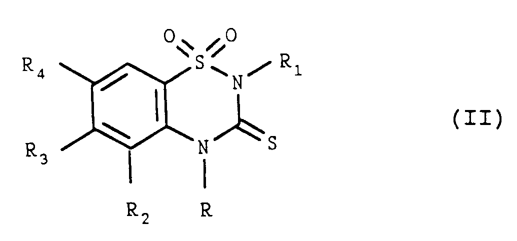
verwendet, in der
- R₁: ein Wasserstoffatom, einen C₁-C₃-Alkylrest oder einen 2-Methylphenylrest,
- R₂: ein Wasserstoff-, Chlor- oder Bromatom,
- R₃: ein Wasserstoff-, Chlor- oder Bromatom oder einen Methoxy-, Methyl- oder Trifluormethylrest und
- R₄: ein Wasserstoff-, Chlor- oder Bromatom oder einen Aminosulfonylrest und
- R: ein Wasserstoffatom, eine C₁-C₆-Alkyl oder Phenyl-C₁-C₆-alkylgruppe bedeuten.

Es wurde nun überraschenderweise gefunden, daß diese 2H-1,2,4-Benzothiazin-3(4H)-thion-1,1-dioxide eine ausgeprägte antivirale Wirkung aufweisen, und sich daher besonders gut zur Behandlung von viralen bzw. retroviralen Infektionen eignen. Virale Infektionen von Säugern, insbesondere des Menschen, sind weit verbreitet. Trotz intensiver Bemühungen ist es bisher nicht gelungen, Chemotherapeutika bereitzustellen, die ursächlich oder symptomatisch mit dem viral oder retroviral bedingten Krankheitsgeschehen mit erkennbar substantiellem Erfolg interferieren. Es ist heutzutage nicht möglich, bestimmte Viruserkrankungen, wie zum Beispiel das Acquired Immune Deficiency Syndrom (AIDS), den AIDS-related-complex (ARC) und deren Vorstadien, Herpes-, Cytomegalie-Virus (CMV)-, Influenza- und andere Virusinfektionen zu heilen oder chemotherapeutisch deren Symptome günstig zu beeinflussen. Derzeit steht beispielsweise für die Behandlung von AIDS fast ausschließlich das 3'-Azido-3'-deoxy-thymidin (AZT), bekannt als Zidovudine oder Retrovir^{R}, zur Verfügung. AZT ist jedoch durch eine sehr enge therapeutische Breite bzw. durch bereits im therapeutischen Bereich auftretende, sehr schwere Toxizitäten charakterisiert (Hirsch, M.S. (1988) J.Infec.Dis. 157, 427-431). Die Verbindungen der allgemeinen Formel I besitzten diese Nachteile nicht. Sie wirken antiviral, ohne in pharmakologisch relevanten Dosen cytotoxisch zu sein.

Die Verbindungen der Formel I und II weisen wertvolle pharmakologische Eigenschaften auf. Insbesondere eignen sie sich zur Therapie und Prophylaxe von Infektionen, die durch DNA-Viren wie z.B. das Herpes-Simplex-Virus, das Zytomegalie-Virus, Papilloma-Viren, das Varicella-Zoster-Virus oder Epstein-Barr-Virus oder RNA-Viren wie Toga-Viren oder insbesondere Retroviren wie die Onko-Viren HTLV-I und II, sowie die Lentiviren Visna und Humanes-Immunschwäche-Virus HIV-1 und -2, verursacht werden.

Besonders geeignet erscheinen die Verbindungen der Formel I und II zur Behandlung der klinischen Manifestationen der retroviralen HIV-Infektion beim Menschen, wie der anhaltenden, generalisierten Lymphadenopathie (PGL), dem fortgeschrittenen Stadium des AIDS-verwandten Komplex (ARC) und dem klinischen Vollbild von AIDS.

Es konnte nun nachgewiesen werden, daß Verbindungen der allgemeinen Formel I und II die Vermehrung von DNA- bzw. RNA-Viren auf der Stufe der virusspezifischen DNA- bzw. RNA-Transkription hemmen. Die Substanzen können über die Inhibierung des Enzyms Reverse Transkriptase die Vermehrung von Retroviren beeinflussen (zur Molekulabiologie von Retroviren vgl. Proc. Natl. Acad. Sci. USA 83, 1911, 1986 bzw. Nature 325, 773, 1987).

Da ein sehr großer Bedarf an Chemotherapeutika besteht, die möglichst spezifisch mit retroviral bedingten Erkrankungen oder deren Symptomen interferieren, ohne die normal ablaufenden natürlichen Körperfunktionen zu beeinflussen, könnten die genannten Verbindungen vorteilhaft prophylaktisch oder therapeutisch bei der Behandlung von Krankheiten eingesetzt werden, bei denen eine retrovirale Infektion von pathophysiologischer, symptomatischer oder klinischer Relevanz ist.

Die Arzneimittel enthaltend Verbindungen der Formel I oder II zur Behandlung von viralen Infektionen können in flüssiger oder fester Form enteral oder parenteral appliziert werden. Hierbei kommen die üblichen Applikationsformen in Frage, wie beispielsweise Tabletten, Kapseln, Dragées, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylen-diamintetraessigsäure und deren nichttoxischen Salze, hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methyl-cellulose, Talkum, hochdisperse Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc. Für orale Applikationen geeignete Zubereitungen können gewünschtenfalls Geschmacks- oder Süßstoffe enthalten.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 - 100 mg, vorzugsweise 0,2 - 80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 500 mg verabreicht werden. Die Tabletten können auch retardiert sein, wodurch sich die Anzahl der Applikationen pro Tag auf 1-3 vermindert. Der Wirkstoffgehalt der retardierten Tabletten kann 2 - 1000 mg betragen. Der Wirkstoff kann auch durch Dauerinfusion gegeben werden, wobei die Mengen von 5 - 1000 mg pro Tag normalerweise ausreichen.

Zur Herstellung der fertigen Darreichungsformen werden die nach an sich bekannten Methoden hergestellten Arzneimittel einzeln konfektioniert und mit dem Hinweis (in der Regel in Form eines Beipackzettels) versehen, daß sich diese Arzneimittel zur Behandlung von viralen oder retroviralen Infektionen eignen.

Die erfindungsgemäß verwendeten Verbindungen der allgemeinen Formel I können nach Vorschriften der im Stand der Technik genannten Patentanmeldungen bzw. Literaturstellen hergestellt werden.

Im Sinne der vorliegenden Erfindung kommen außer den durch Kombinaton aller Ansprüche möglichen Verbindungen, besonders die nachfolgend genannten Derivate in Frage.
1. 2-Ethyl-2H-1,2-4-benzothiadiazin-3(4H)-thion-1,1-dioxid Schmp. 143° C
2. 5-Chlor-2-ethyl-2H-1,2-4-benzothiadiazin-3(4H)-thion-1,1-dioxid, Schmp. 125-126° C
3. 6-Methyl-2-ethyl-2H-1,2-4-benzothiadiazin-3(4H)-thion-1,1-dioxid, Schmp. 176-177° C
4. 6-Chlor-2-ethyl-2H-1,2-4-benzothiadiazin-3(4H)-thion-1,1-dioxid, Schmp. 176-177° C
5. 6-Methoxy-2-ethyl-2H-1,2-4-benzothiadiazin-3(4H)-thion-1,1-dioxid, Schmp. 172-173° C
6. 5,7-Dichlor-2-ethyl-2H-1,2-4-benzothiadiazin-3(4H)-thion-1,1-dioxid
7. 5,7-Dibrom-2-ethyl-2H-1,2-4-benzothiadiazin-3(4H)-thion-1,1-dioxid
8. 7-Chlor-2-ethyl-2H-1,2-4-benzothiadiazin-3(4H)-thion-1,1-dioxid, Schmp. 205-209° C
9. 7-Brom-2-ethyl-2H-1,2-4-benzothiadiazin-3(4H)-thion-1,1-dioxid
10. 6-Chlor-7-sulfonamido-2-ethyl-2H-1,2-4-benzothiadiazin-3(4H)-thion-1,1-dioxid
11. 6-Trifluormethyl-7-sulfonamido-2-ethyl-2H-1,2-4-benzothiadiazin-3(4H)-thion-1,1-dioxid
12. 2H-1,2,4-benzothiadiazin-3(4H)-thion-1,1-dioxid, Schmp. 220° C
13. 2-Methyl-2H-1,2,4-benzothiadiazin-3(4H)-thion-1,1-dioxid, Schmp. 194° C
14. 2-Propyl-2H-1,2,4-benzothiadiazin-3(4H)-thion-1,1-dioxid
15. 7-Chlor-2-propyl-2H-1,2,4-benzothiadiazin-3(4H)-thion-1,1-dioxid
16. 2,6-Dimethyl-2H-1,2,4-benzothiadiazin-3(4H)-thion-1,1-dioxid
17. 5-Chlor-2-propyl-2H-1,2,4-benzothiadiazin-3(4H)-thion-1,1-dioxid
18. 7-Methyl-2-ethyl-2H-1,2,4-benzothiadiazin-3(4H)-thion-1,1-dioxid, Schmp. 172-177° C
19. 5-Methyl-2-ethyl-2H-1,2,4-benzothiadiazin-3(4H)-thion-1,1-dioxid, Schmp. 167-171° C
20. 5-Methoxy-2-ethyl-2H-1,2,4-benzothiadiazin-3(4H)thion-1,1-dioxid, Schmp. 173-174° C
21. 2-Isopropyl-2H-1,2,4-benzothiadiazin-3(4H)thion-1,1-dioxid, Schmp. 161° C
22. 2-Phenyl-2H-1,2,4-benzothiadiazin-3(4H)thion-1,1-dioxid, Schmp. 210-211° C
23. 2-Isobutyl-2H-1,2,4-benzothiadiazin-3(4H)thion-1,1-dioxid, Schmp. 145-146° C
24. 8-Methoxy-2-ethyl-2H-1,2,4-benzothiadiazin-3(4H)thion-1,1-dioxid, Schmp. 198-200° C
25. 2-Ethyl-4-benzyl-2H-1,2,4-benzothiadiazin-3(4H)thion-1,1-dioxid, Schmp. 100-103° C

### RT-Test

Das Screeningtestsystem beinhaltet die gereinigte RT (Reverse Transkriptase) aus HIV-1, die durch gentechnologische Methoden in E. coli exprimiert wurde, sowie die Komponenten des Initiationskomplexes, wie die in-vitro-Transkripte des HIV-LTR's mit der benachbarten Primer Binding Site als Template und einem zur Primer Binding Site komplementären 18mer Oligonukleotid als Primer. Gemessen wurde der [³H]-Thymidin-5'-triphosphat-Einbau durch Auszählen im β-Counter.

| Ergebnisse | |
|---|---|
| Substanz | Hemmung der HIV-RT IC₅₀[M] |
| 2-Ethyl-2H-1,2,4-benzothiadiazin-3(4H)-thion-1,1-dioxid | 8.3 x 10⁻⁶ |
| 7-Chlor-2-ethyl-2H-1,2,4-benzothiadiazin-3(4H)-thion-1,1-dioxid | 1.6 x 10⁻⁶ |
| 2-Isopropyl-2H-1,2,4-benzothiadiazin-3(4H)-thion-1,1-dioxid | 1.6 x 10⁻⁵ |

## Patentansprüche

1. Verwendung von 2H-1,2,4-Benzothiadiazin-3(4H)-thion-1,1-dioxiden der Formel I 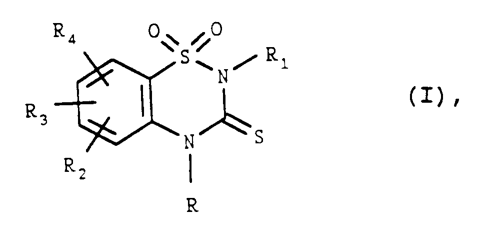 in der
R₁ ein Wasserstoffatom, einen C₁-C₆-Alkylrest oder einen gegebenenfalls durch C₁-C₆-Alkyl substituierten Phenylrest bedeutet,
R₂-R₄ gleich oder verschieden sind und unabhängig voneinander ein Wasserstoff- oder Halogenatom, einen C₁-C₆-Alkyl-, C₁-C₆-Alkoxy-, Trifluormethyl- oder Aminosulfonylrest und
R ein Wasserstoffatom, eine C₁-C₆-Alkyl- oder Phenyl-C₁-C₆-alkylgruppe bedeuten,
oder deren Tautomere zur Herstellung von Arzneimitteln zur Behandlung von viralen oder retroviralen Infektionen.

2. Verwendung gemäß Anspruch 1, dadurch gekennzeichnet, daß die 2H-1,2,4-Benzothiadiazin-3(4H)-thion-1,1-dioxide der allgemeinen Formel II 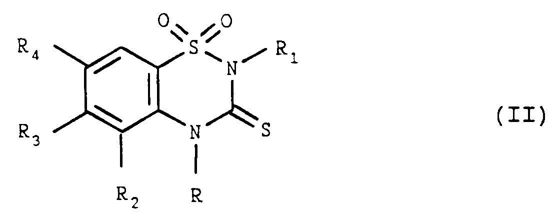 entsprechen, in der
R₁ ein Wasserstoffatom, einen C₁-C₃-Alkylrest oder einen 2-Methylphenylrest,
R₂ ein Wasserstoff-, Chlor- oder Bromatom,
R₃ ein Wasserstoff-, Chlor- oder Bromatom oder einen Methoxy-, Methyl- oder Trifluormethylrest und
R₄ ein Wasserstoff-, Chlor- oder Bromatom oder einen Aminosulfonylrest und
R ein Wasserstoffatom, eine C₁-C₆-Alkyl -oder Phenyl-C₁-C₆-alkylgruppe bedeuten.

## Claims

1. Use of 2H-1,2,4-benzothiadiazine-3(4H)-thione 1,1-dioxides of the formula I 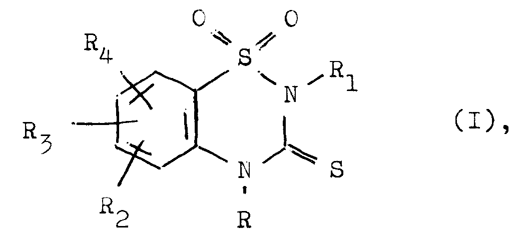 in which R₁ signifies a hydrogen atom, a C₁-C₆-alkyl radical or a phenyl radical possibly substituted by C₁-C₆-alkyl, R₂ - R₄ are the same or different and, independently of one another, signify a hydrogen or halogen atom, a C₁-C₆-alkyl, C₁-C₆-alkoxy, trifluoromethyl or aminosulphonyl radical and R a hydrogen atom, a C₁-C₆-alkyl or phenyl-C₁-C₆-alkyl group, or of their tautomers for the production of medicaments for the treatment of viral or retroviral infections.

2. Use according to claim 1, characterised in that the 2H-1,2,4-benzothiadiazine-3(4H)-thione-1,1-dioxides correspond to the general formula II 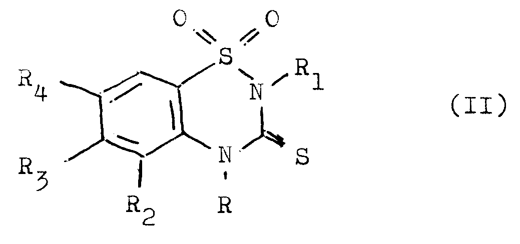 in which R₁ signifies a hydrogen atom, a C₁-C₃-alkyl radical or a 2-methylphenyl radical, R₂ a hydrogen, chlorine or bromine atom, R₃ a hydrogen, chlorine or bromine atom or a methoxy, methyl or trifluoromethyl radical and R₄ a hydrogen, chlorine or bromine atom or an aminosulphonyl radical and R a hydrogen atom, a C₁-C₆-alkyl or phenyl-C₁-C₆-alkyl group.

## Revendications

1. Utilisation de 1,1-dioxydes de 2H-1,2,4-benzothiadiazine-3(4H)-thiones, de formule 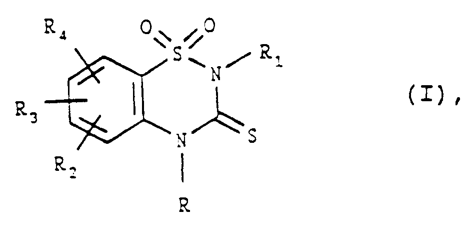 dans laquelle
R₁ représente un atome d'hydrogène, un reste alkyle en C₁-C₆ ou un reste phényle éventuellement substitué par un reste alkyle en C₁-C₆,
R₂-R₄ sont identiques ou différents et représentent indépendamment un atome d'hydrogène ou d'halogène, un reste alkyle en C₁-C₆, alcoxy en C₁-C₆, trifluorométhyle ou aminosulfonyle, et
R représente un atome d'hydrogène, un reste alkyle en C₁-C₆ ou phényl-(alkyle en C₁-C₆),
ou de leurs tautomères, pour la préparation de médicaments destinés au traitement d'infections à virus ou rétrovirus.

2. Utilisation selon la revendication 1, caractérisée en ce que les 1,1-dioxydes de 2H-1,2,4-benzothiadiazine-3(4H)-thiones répondent à la formule générale II 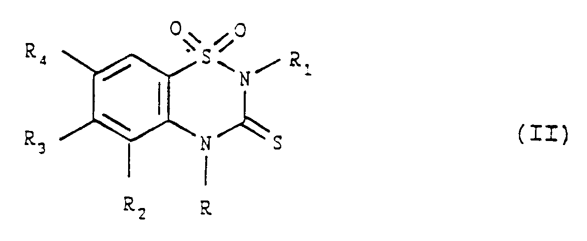 dans laquelle
R₁ représente un atome d'hydrogène, un reste alkyle en C₁-C₃ ou un reste 2-méthylphényle,
R₂ représente un atome d'hydrogène, de chlore ou de brome,
R₃ représente un atome d'hydrogène, de chlore ou de brome ou un reste méthoxy, méthyle ou trifluorométhyle, et
R₄ représente un atome d'hydrogène, de chlore ou de brome ou un reste aminosulfonyle, et
R représente un reste alkyle en C₁-C₆ ou phényl-(alkyle en C₁-C₆).
